# EUROPÄISCHE PATENTSCHRIFT

(11) **EP 1 245 204 B1**
(45) Veröffentlichungstag und Bekanntmachung des Hinweises auf die Patenterteilung: **07.02.2007**
(21) Anmeldenummer: 01811196.3
(22) Anmeldetag: 07.12.2001
(51) Int. Cl.: A61F 2/38

(54) **Knieprothese**
Knee prosthesis
Prothèse de genou

(30) Priorität: 26.03.2001 EP 01810301
(43) Veröffentlichungstag der Anmeldung: 02.10.2002
(73) Patentinhaber: Zimmer GmbH, 8404 Winterthur (CH)
(72) Erfinder: Leclercq, Vincent, 8404 Winterthur (CH)
(74) Vertreter: Manitz, Finsterwald & Partner GbR

(56) Entgegenhaltungen:
- EP-A- 0 519 873
- FR-A- 2 682 287
- FR-A- 2 768 329

## Beschreibung

Die Erfindung handelt von einer Knieprothese mit einem Femurteil, welcher Führungsflächen für eine Patella und mindestens eine Kondyle aufweist, welcher äussere Anlageflächen in einem spitzen Winkel α ≤ 90° besitzt und welcher entlang einer Geraden im Schwenkbereich des Winkels α mit Anlageflächen auf Resektionsflächen am Stumpf eines Femurknochens aufschiebbar ist. Dokument FR-A-2768329 offenbart die Merkmale des Oberbegriffs von Anspruch 1.

Bisher ist es üblich, bei einer Knieprothese die Kondylen in Form eines nach oben geöffneten "U" auf einen vorbearbeiteten Femurknochen aufzuschieben und zu befestigen. Dazu wird der Femurstumpf im Bereich der natürlichen Kondylen auf die Form der Anlageflächen der künstlichen Kondylenteile gebracht, um diese anschliessend zu befestigen. Die Befestigung selbst kann durch vorstehende Zapfen an den Anlageflächen, durch ein Verkeilen der äusseren, sich gegenüberliegenden Anlageflächen und / oder durch ein Fixieren der Anlageflächen mit Knochenzement vorgenommen werden. Die Kondylen liegen auf einem Meniskusteil oder auf einer Plattform auf und können während der Artikulation des Kniegelenkes relativ zur Plattform respektive zur Tibia auf ihren Laufflächen um einen Flexionswinkel δ geschwenkt werden.

In der EP-A-0 519 873 sind künstliche Kniegelenke gezeigt, bei denen je nach dem Zustand der Bänder auch eine mehr oder weniger wirksame seitliche Führung der Kondylen während der Artikulation möglich ist.

Die obere Prothesenhälfte besitzt Führungsflächen für eine Patella, die gegen anterior angeordnet ist und während der Flexion gleitet die Patella, welche um etwa 90° zur Plattform versetzt ist, auf eigenen Führungsflächen an der oberen Prothesenhälfte. Die Auflageflächen der oberen Prothesenhälfte bilden eine "U"-förmige Öffnung gegen oben, wobei in der Extension die Patella am vorderen Schenkel und die Plattform am Boden dieses "U" anliegen, während bei der Flexion der Boden unter die Patella und der hintere Schenkel zur Plattform dreht. Am Femurstumpf treten sowohl Kräfte von der Patella von vorn als auch Kräfte von hinten bei Flexion von der Plattform auf, die durch gegenüberliegende, annähernd zueinander senkrecht stehende Resektionsflächen abgefangen werden. Bei den meisten oberen Prothesenteilen stehen die zugehörigen äusseren Anlageflächen in einem kleinen Winkel zueinander, damit beim Aufschieben durch die Keilwirkung ein fester Sitz zum Prothesenstumpf erreicht wird. Für viele Bewohner im asiatischen Raum ist der mit einer solchen Prothese erreichbare Flexionswinkel ungenügend, da sie von Natur aus mit einem grösseren Flexionswinkel zu leben gewohnt sind und eine Knieprothese in Flexionswinkel bringen, für die letztere nicht geeignet sind.

Im FR-A-2768329 weist der Femurteil ein Mittelteil mit Führungsflächen für die Patella und ein davon getrennt aufschiebbares Kondylenteil auf, welches äussere Anlageflächen in einem spitzen Winkel β ≤ 90° besitzt und welches entlang einer Geraden im Schwenkbereich des Winkels β auf Resektionsflächen am Stumpf des Femurknochens aufschiebbar ist. Aufgabe der Erfindung ist es, Konstruktionsformen aufzuzeigen, die bei einem offenen Gelenk grosse Flexionswinkel ermöglichen. Diese Aufgabe wird dadurch gelöst, dass die Mittellinie des Winkels β am Kondylenteil gegenüber der Mittellinie des Winkels α am Mittelteil um einen Winkel 15° ≤ γ ≤ 60° verschwenkt ist, um eine grössere Flexion zu ermöglichen.

Der Vorteil der Erfindung liegt darin, dass auch bei grosser Flexion die Kräfte, welche durch das Prothesenoberteil auf den Prothesenstumpf übertragen werden, Druckkräfte sind. Es treten keine Scherbelastungen zwischen Anlageflächen und Resektionsflächen auf, die entgegen der Aufschieberichtung sind. Dadurch, dass der Prothesenoberteil in ein Mittelteil und in ein Kondylenteil aufgeteilt ist, deren Trennfuge so verläuft, dass die Führungsflächen für die Patella im Mittelteil angeordnet sind und Laufflächen zur Plattform auf dem Kondylenteil angeordnet sind, können diese Flächen unabhängig voneinander gefertigt werden und unabhängig voneinander in zueinander verschwenkten Aufschieberichtungen auf den Femurstumpf aufgeschoben werden. Trotz unterschiedlicher Aufschieberichtungen können Anlageflächen von Mittelteil und von Kondylenteil gemeinsam an einer Resektionsfläche anliegen. Die Aufschieberichtungen sind beispielsweise um einen Winkel γ zwischen 15° und 60° verschwenkt, um einen grossen Flexionswinkel δ zu ermöglichen. Dabei sind Flexionswinkel δ von mehr als 120° beispielsweise 160° möglich. Die Kondylen können mit einem Joch verbunden sein oder einzeln ausgeführt sein. Im aufgeschobenen Zustand sind die Kondylen durch die Resektionsflächen, d. h. über den Femurstumpf zum Mittelteil ausgerichtet. Zusätzlich können Mittelteil und Kondylenteile in sagittalen Ebenen zueinander beweglich geführt sein, um am Femurstumpf ähnliche Kraftangriffspunkte von Patella und Plattform wie bei einem natürlichen Kniegelenk zu erhalten. Es besteht aber auch die Möglichkeit Mittelteil und Kondylen durch ein Kupplungsorgan im aufgeschobenen Zustand zu verbinden.

Für eine Befestigung mit Knochenzement können einzelne Anlageflächen Taschen aufweisen um eine Mindestmenge an Knochenzement einzubringen. Einzelne Anlageflächen insbesondere die äusseren Anlageflächen können eine gezahnte Verankerungsstruktur für eine Primärverankerung aufweisen. Ebenso können einzelne Zapfen, die in Aufschieberichtung verlaufen und für die in Aufschieberichtung Bohrungen vorgesehen sind, die Primärverankerung verbessern. Die nicht mit Knochenzement benetzten Anlageflächen können mit einem Belag beschichtet sein, der das Knochenwachstum fördert.

Anlageflächen, die von Knochenzement benetzt werden, können mit einem Haftvermittler beschichtet sein. So ein Belag kann beispielsweise aus einem Titanalalkoholat und einem Alkoxysilan aufgebaut sein.

Während der Flexion kann sich der im Eingriff stehende Krümmungsradius der Kondylen kontinuierlich oder sprungweise verringern, um mit zunehmender Flexion eine seitliche Auslenkung der Kondylen zu ermöglichen.

Im folgenden wird die Erfindung anhand von Ausführungsbeispielen beschrieben. Es zeigen:
- Fig. 1:: schematisch einen Femurstumpf mit vorbearbeiteten Resektionsflächen, auf den ein gemäss
- Fig. 2:: schematisch gezeigtes Mittelteil entlang einer Mittellinie aufschiebbar ist und ein gemäss
- Fig. 3:: schematisch gezeigtes Kondylenteil entlang einer um einen Winkel γ verschwenkten Mittellinie aufschiebbar ist;
- Fig. 4:: schematisch eine Ansicht für ein weiteres Beispiel von einem Mittelteil;
- Fig. 5:: schematisch ein zu Figur 4 passendes Kondylenteil mit einem Joch;
- Fig. 6:: schematisch eine Seitenansicht von Figur 4;
- Fig. 7:: schematisch eine Seitenansicht von Figur 5;
- Fig. 8:: schematisch eine Ansicht von unten der Figuren 4 und 5 vor dem Aufschieben
- Fig. 9:: schematisch Mittelteil und Kondylenteil von Figur 8 nach dem Aufschieben
- Fig. 10:: schematisch eine Ansicht von einem Mittelteil mit zwei voneinander unabhängigen Kondylen vor dem Aufschieben;
- Fig. 11:: schematisch unter einem etwas anderen Blickwinkel die Teile von Figur 10 nach dem Aufschieben; und
- Fig. 12:: schematisch einen vergrösserten Ausschnitt einer äusseren Anlagefläche mit einer Zahnung für die Primärverankerung.

In den Figuren sind Femurteile von Knieprothesen gezeigt, die ein Mittelteil 2 und ein Kondylenteil 3 mit Anlageflächen 4a, 5a, 6a, 6b, 7b, 8a, 9a, 10a, 11a, 11b, 12a, 12b, 13b zu einem reserzierten Femurstumpf 1 besitzen. Dabei besitzt der Mittelteil 2 Führungsflächen 20 für eine Patella 19 und äussere Anlageflächen 4a, 5a die in einem spitzen Winkel α ≤ 90° zueinander stehen, während der Kondylenteil 3 Laufflächen 27, 28 für die Artikulationsbewegung und äussere Anlageflächen 6b, 7b in einem spitzen Winkel β ≤ 90° zueinander besitzt. Mittelteil 2 und Kondylenteil 3 sind getrennt voneinander auf den Femurstumpf in zueinander verschwenkten Aufsteckrichtungen aufsteckbar um eine grössere Flexion zu ermöglichen, wobei die Mittellinie des Winkels β gegenüber der Mittellinie des Winkels α um einen Winkel 15° ≤ γ ≤ 60° verschwenkt ist.

Im folgenden sind für gleiche Funktionen gleiche Hinweiszeichen verwendet.

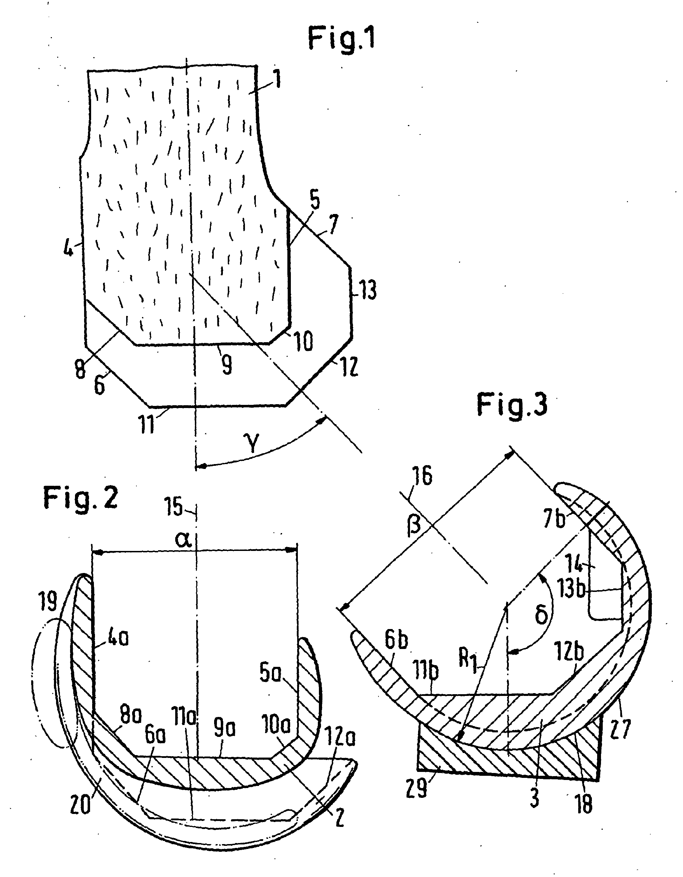

Im Beispiel der Figuren 1,2 und 3 sind an einem Femurstumpf 1 im Bereich der Kondylen Resektionsflächen 4, 6, 11, 12, 12, 13, 7 und im Bereich zwischen den Kondylen Resektionsfiächen 4, 8, 9, 10, 5 angebracht. Die Resektionsflächen 4 und 5 sowie die daran anliegenden äusseren Anlageflächen 4a, 5a des Mittelteils 2 stehen in einem spitzen Winkel fast parallel zueinander. Das Mittelteil stützt sich mit den Anlageflächen 8a, 9a, 10a zwischen den Kondylen und mit weiteren Anlageflächen 6a, 11a, 12a auf Resektionsflächen im Bereich der früheren, natürlichen Kondylen ab, wobei diese Resektionsflächen auch von Anlageflächen 6b, 11b, 12b des Kondylenteils 3 belegt werden, um eine Zentrierung im Graben zwischen den Kondylen vorzunehmen und um den Führungsflächen 20 für die Patella 19 auch bei vollständiger Flexion Unterstützung zu geben.

Der Mittelteil ist entlang der Mittellinie 15 des Winkels α von unten aufsteckbar, während die Aufsteckrichtung und die Mittellinie 16 des Winkels β um einen Winkel γ von 45° verschwenkt ist um auch bei einem grösseren Flexionswinkel δ, beispielsweise δ > 120°, eine äussere Anlagefläche 7b zu schaffen, bei der die Kontaktkraft die Fläche 7b in den Stumpf hinein oder höchstens senkrecht zur Fläche 7b presst. Wie aus Figur 3 ersichtlich ist, haben die Lauffläche 27 des Kondylenteils 3 und die Lauffläche 18 eines Meniskusteils 18 den gleichen Krümmungsradius R₁.

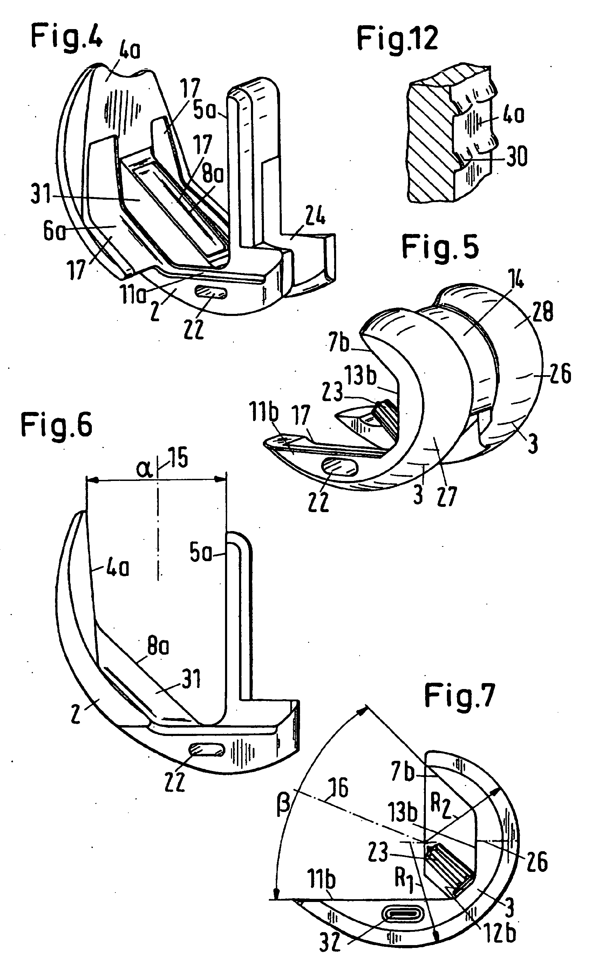

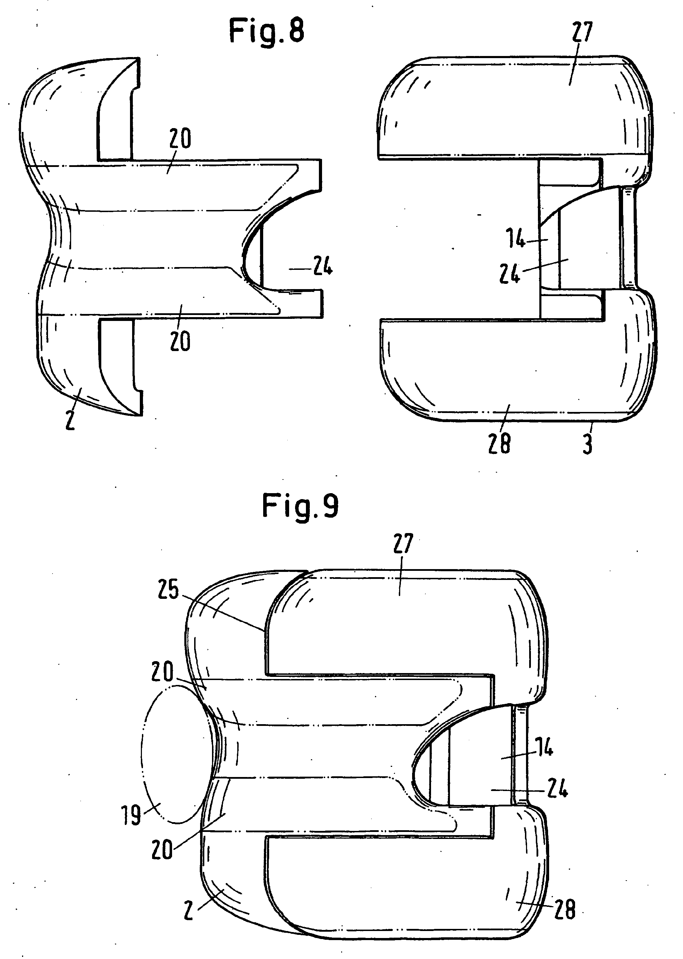

Ein weiteres Beispiel ist in den Figuren 4, 5, 6, 7, 8, 9 gezeigt. Auch hier müssen wegen des vergrösserten Flexionswinkels die Führungsflächen 20 für die Patella 19 und die Laufflächen 27, 28 in Flexionsrichtung verlängert werden. Der Mittelteil 2 besitzt Anlageflächen 4a, 6a, 8a, 11a, 5a und seitliche Zentrierflächen 31. Der Kondylenteil 2 besitzt ein Joch 14 welches die eigentlichen Kondylen verbindet. Eine Trennfuge 25 zwischen dem Mittelteil 2 und dem Kondylenteil 3 ist so gelegt (Fig. 8, 9), dass die Führungsflächen 20 für die Patella 19 und die Laufflächen 27, 28 der Kondylen nicht unterbrochen sind. Eine Aussparung 24 ist für Kreuzbänder vorgesehen. Ein Teil der Anlageflächen 4a, 6a, 8a, 11a besitzt Taschen 17, die beim Aufschieben mit Knochenzement gefüllt werden, um eine Mindestschichtdicke an Knochenzement in bestimmten Bereichen zu ermöglichen. Die Taschen 17 und Anlageflächen können auch mit einem Haftvermittler und einer dünnen Schicht aus Knochenzement vorzementiert sein.

Gemäss dem spitzen Winkel α in Figur 6 muss das Aufschieben des Mittelteils 2 in etwa in der Richtung der Mittellinie 15 erfolgen, während beim Kondylenteil mit einem weniger spitzen Winkel β eine grössere Abweichung der Aufschieberichtung von der Mittellinie des Winkels β möglich ist. Hier wird die Aufschieberichtung, die innerhalb des Winkels β liegen muss, durch die Richtung der Zapfen 23 bestimmt. In Figur 5 und 7 ist eine Erzeugende für die Kontur der Kondylenlaufflächen 27, 28 angedeutet. Der Krümmungsradius R₁ der Laufflächen 27, 28 geht mit zunehmender Flexion in einen kleineren Krümmungsradius R₂ über.

Mittelteil 2 und Kondylenteil 3 besitzen Bohrungen 22, damit sie nach dem Aufstecken auf den Femurstumpf mit einem Kupplungsstück in Form eines Stiftes 32 verbunden werden können. Durch diesen Verbund ist der Femurstumpf gleichzeitig gefangen, da er auf einem Umschlingungswinkel von mehr als 180° von Anlageflächen umschlossen ist.

In Figur 12 zeigt der Ausschnitt der Anlagefläche 4a, dass es im Fall von äusseren Anlageflächen, die in einem sehr spitzen Winkel wie beispielsweise in Figur 6 die Flächen 4a und 5a zueinanderstehen, sinnvoll sein kann, diese Fläche mit einer Zahnung zu versehen, die eine gute Primärverankerung ermöglicht und den Mittelteil 2 sichert, bis auch der Kondylenteil 3 aufgesteckt ist.

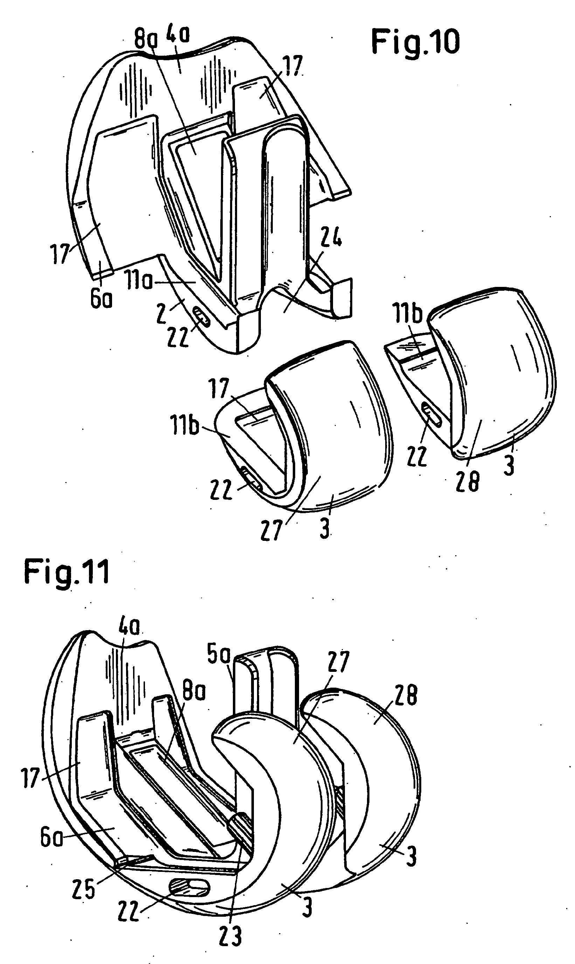

Am Beispiel der Figuren 10 und 11 ist eine Anordnung gezeigt, bei der zwei separate Kondylenteile 3 mit Laufflächen 27, 28 aufsteckbar sind und über Bohrungen 22 durch ein Kupplungsstück, beispielsweise einen Stift oder eine Schraube, mit dem Mittelteil 2 verbunden werden können. Wenn es der Bandapparat am Knie erlaubt, können diese beiden Kondylenteile 3 auch von der Seite her eingebracht werden, da sie nicht fest mit einem Joch verbunden sind, um dann - je nachdem wie lang die Zapfen 23 gestaltet sind - in der Aufschieberichtung der Zapfen ihre Verankerung zu finden. Sobald ein Kondylenteil 3 mit dem Mittelteil 2 verbunden ist, wird auch hier der Femurstumpf auf einem Umschlingungswinkel von mehr als 180° umschlossen und ist ohne Risiko ein Flexionswinkel von mehr als 120° möglich.

## Patentansprüche

1. Knieprothese mit einem Femurteil, welcher Führungsflächen (20) für eine Patella (19) und mindestens eine Kondyle (18) aufweist, welcher äussere Anlageflächen (4a, 5a) in einem spitzen Winkel α ≤ 90° besitzt und welcher entlang einer Geraden im Schwenkbereich des Winkels α mit Anlageflächen auf Resektionsflächen (4, 5, 6, 8, 9, 10, 11, 12) am Stumpf eines Femurknochens (1) aufschiebbar ist, wobei der Femurteil ein Mittelteil (2) mit Führungsflächen (20) für die Patella (19) und ein davon getrennt aufschiebbares Kondylenteil (3) aufweist, welches äussere Anlageflächen (6b, 7b) in einem spitzen Winkel β ≤ 90° besitzt und welches entlang einer Geraden im Schwenkbereich des Winkels β auf Resektionsflächen (6, 7, 11, 12, 13) am Stumpf des Femurknochens aufschiebbar ist, **dadurch gekennzeichnet, dass** die Mittellinie (16) des Winkels β am Kondylenteil (3) gegenüber der Mittellinie (15) des Winkels α am Mittelteil (2) um einen Winkel 15° ≤ γ ≤ 60° verschwenkt ist, um eine grössere Flexion zu ermöglichen.

2. Knieprothese nach Anspruch 1, **dadurch gekennzeichnet, dass** der Kondylenteil (3) einen Flexionswinkel δ von mehr als 120° aufweist.

3. Knieprothese nach einem der Ansprüche 1 oder 2, **dadurch gekennzeichnet, dass** der Kondylenteil (3) zwei jeweils zum Mittelteil (2) aussenliegende Kondylen (3) aufweist, die über ein Joch (14) verbunden sind.

4. Knieprothese nach einem der Ansprüche 1 bis 3, **dadurch gekennzeichnet, dass** Mittelteil (2) und Kondylenteil (3) im aufgeschobenen Zustand durch ein Kupplungsorgan (21) miteinander verbindbar sind.

5. Knieprothese nach Anspruch 4, **dadurch gekennzeichnet, dass** das Kupplungsorgan (21) aus einem an Trennflächen quer durchgesteckten Stift oder aus einer quer durchgehenden Verschraubung besteht.

6. Knieprothese nach einem der Ansprüche 1 bis 5, **dadurch gekennzeichnet, dass** der Winkel γ zwischen 40° und 50° liegt.

7. Knieprothese nach einem der Ansprüche 1 bis 6, **dadurch gekennzeichnet, dass** einzelne Anlageflächen (4a, 5a, 6a, 6b, 7b, 8a, 9a, 10a, 11a, 11b, 12a, 12b, 13b) Taschen (17) aufweisen, um für eine Befestigung mit Knochenzement eine Mindestmenge an Knochenzement einzubringen.

8. Knieprothese nach einem der Ansprüche 1 bis 6, **dadurch gekennzeichnet, dass** einzelne Anlageflächen (4a, 5a, 6a, 6b, 7b, 8a, 9a, 10a, 11a, 11 b, 12a, 12b, 13b) eine gezahnte Verankerungsstruktur (30) für eine Primärverankerung aufweisen.

9. Knieprothese nach einem der Ansprüche 1 bis 6, **dadurch gekennzeichnet, dass** einzelne Anlageflächen (4a, 5a, 6a, 6b, 7b, 8a, 9a, 10a, 11a, 11 b, 12a, 12b, 13b) mit einem Belag beschichtet sind, der das Knochenwachstum fördert oder die Haftung von Knochenzement verbessert.

10. Knieprothese nach Anspruch 9, **dadurch gekennzeichnet, dass** der Belag Stoffe wie Hydroxylappatit, enthält, oder aus einem Titanalalkoholat und einem Alkoxysilan aufgebaut ist.

11. Knieprothese nach einem der Ansprüche 1 bis 10, **dadurch gekennzeichnet, dass** sich der Krümmungsradius R₁ der Kondylen (3) im Auflagebereich vom Übergang der Extension bis zur vollen Flexion mindestens einmal verringert.

12. Knieprothese nach einem der Ansprüche 1 bis 11, **dadurch gekennzeichnet, dass** der Winkel α oder β kleiner 10° ist.

13. Knieprothese nach einem der Ansprüche 1 bis 11, **dadurch gekennzeichnet, dass** der Winkel α oder β kleiner 5° ist.

14. Knieprothese nach einem der Ansprüche 1 bis 13, **dadurch gekennzeichnet, dass** an Anlageflächen (12b) in Aufschieberichtung Zapfen (23) angebracht sind, die die Zentrierung und Primärverankerung erleichtern.

## Claims

1. A knee prosthesis having a femur part which has guide surfaces (20) for a patella (19) and at least one condyle (18), which has outer contact surfaces (4a, 5a) at an acute angle α ≤ 90° and whose contact surfaces can be pushed onto resection areas (4, 5, 6, 8, 9, 10, 11, 12) at the stump of a femur bone (1) along a straight line in the pivot range of the angle α, wherein the femur part has a centre part (2) with guide surfaces (20) for the patella (19) and a condyle part (3) which can be pushed on separately therefrom, which has outer contact surfaces (6b, 7b) at an acute angle β ≤ 90° and which can be pushed onto resection areas (6, 7, 11, 12, 13) at the stump of the femur bone along a straight line in the pivot range of the angle β, **characterised in that** the centre line (16) of the angle β at the condyle part (3) is pivoted through an angle of 15° ≤ γ ≤ 60° with respect to the centre line (15) of the angle α at the centre part (2) in order to allow a greater flexion.

2. A knee prosthesis in accordance with claim 1, **characterised in that** the condyle part (3) has a flexion angle δ of more than 120°.

3. A knee prosthesis in accordance with claim 1 or claim 2, **characterised in that** the condyle part (3) has two condyles (3) each outwardly disposed with respect to the centre part (2) which are connected via a yoke (14).

4. A knee prosthesis in accordance with any of claims 1 to 3, **characterised in that** the centre part (2) and the condyle part (3) are connectable to one another by a coupling member (21) in the pushed-on state.

5. A knee prosthesis in accordance with claim 4, **characterised in that** the coupling member (21) consists of a pin inserted transversely through separating surfaces or of a transversely throughgoing screw connection.

6. A knee prosthesis in accordance with any one of claims 1 to 5, **characterised in that** the angle γ is between 40° and 50°.

7. A knee prosthesis in accordance with any of claims 1 to 6, **characterised in that** individual contact surfaces (4a, 5a, 6a, 6b, 7b, 8a, 9a, 10a, 11a, 11b, 12a, 12b, 13b) have pockets (17) in order to introduce a minimum quantity of bone cement for a fastening with bone cement.

8. A knee prosthesis in accordance with any one of claims 1 to 6, **characterised in that** individual contact surfaces (4a, 5a, 6a, 6b, 7b, 8a, 9a, 10a, 11a, 11b, 12a, 12b, 13b) have a serrated anchoring structure (30) for a primary anchoring.

9. A knee prosthesis in accordance with any of claims 1 to 6, **characterised in that** individual contact surfaces (4a, 5a, 6a, 6b, 7b, 8a, 9a, 10a, 11a, 11b, 12a, 12b, 13b) are covered with a coating which promotes bone growth or enhances the adhesion of bone cement.

10. A knee prosthesis in accordance with claim 9, **characterised in that** the film contains substances such as hydroxyl appatite or is made up of a titanal alcoholate and an alkoxy silane.

11. A knee prosthesis in accordance with any of claims 1 to 10, **characterised in that** the radius of curvature R₁ of the condyles (3) is reduced at least once in the contact region from the transition of the extension to the full flexion.

12. A knee prosthesis in accordance with any of claims 1 to 11, **characterised in that** the angle α or β is less than 10°.

13. A knee prosthesis in accordance with any of claims 1 to 11, **characterised in that** the angle α or β is less than 5°.

14. A knee prosthesis in accordance with any of claims 1 to 13, **characterised in that** spigots (23) are attached to contact surfaces (12b) in the direction of pushing on which facilitate centering and primary anchoring.

## Revendications

1. Prothèse de genou, comprenant une partie côté fémur qui présente des surfaces de guidage (20) pour une patella (19) et au moins un condyle (18), qui possède des surfaces d'appui extérieures (4a, 5a) sous un angle aigu α ≤ 90°, et qui peut être enfilé le long d'une droite dans la plage de pivotement de l'angle α, au moyen de surfaces d'appui sur des surfaces de résection (4, 5, 6, 8, 9, 10, 11, 12) sur le moignon d'un os du fémur (11), ladite partie côté fémur comprenant une partie médiane (2) avec des surfaces de guidage (20) pour la patella (19) et une partie de condyle (3) susceptible d'être enfilée et séparée de celle-ci, qui possède des surfaces d'appui (6b, 7b) sous un angle aigu β≤ 90°, et est susceptible d'être enfilée le long d'une droite dans la plage de pivotement de l'angle β sur des surfaces de résection (6, 7, 11, 12, 13) sur le moignon de l'os de fémur, **caractérisée en ce que** la ligne médiane (16) de l'angle β sur la partie de condyle (3) est pivotée d'un angle 15° ≤ γ ≤ 60° par rapport à la ligne médiane (15) de l'angle α sur la partie médiane (2) pour permettre une flexion plus importante.

2. Prothèse de genou selon la revendication 1, **caractérisée en ce que** la partie de condyle (3) présente un angle de flexion δ supérieur à 120°.

3. Prothèse de genou selon l'une des revendications 1 ou 2, **caractérisée en ce que** la partie de condyle (3) comporte deux condyles (3) situés respectivement à l'extérieur par rapport à la partie médiane (2), qui sont reliés via une traverse (14).

4. Prothèse de genou selon l'une des revendications 1 à 3, **caractérisée en ce que** la partie médiane (2) et la partie de condyle (3) sont susceptibles d'être reliées dans l'état enfilé au moyen d'un organe de couplage (21).

5. Prothèse de genou selon la revendication 4, **caractérisée en ce que** l'organe de couplage (21) est formé par une tige enfoncée transversalement au niveau des surfaces de séparation ou par un vissage traversant transversal.

6. Prothèse de genou selon l'une des revendications 1 à 5, **caractérisée en ce que** l'angle γ est entre 40° et 50°.

7. Prothèse de genou selon l'une des revendications 1 à 6, **caractérisée en ce que** des surfaces d'appui individuelles (4a, 5a, 6a, 6b, 7b, 8a, 9a, 10a, 11a, 11b, 12a, 12b, 13b) comportent des poches (17) pour introduire une quantité minimum de ciment osseux pour une fixation au moyen d'un ciment osseux.

8. Prothèse de genou selon l'une des revendications 1 à 6, **caractérisée en ce que** des surfaces d'appui individuelles (4a, 5a, 6a, 6b, 7b, 8a, 9a, 10a, 11a, 11b, 12a, 12b, 13b) comportent une structure d'ancrage dentée (30) pour un ancrage primaire.

9. Prothèse de genou selon l'une des revendications 1 à 6, **caractérisée en ce que** des surfaces d'appui individuelles (4a, 5a, 6a, 6b, 7b, 8a, 9a, 10a, 11a, 11b, 12a, 12b, 13b) sont enduites d'un revêtement qui favorise la croissance osseuse ou qui améliore l'adhérence du ciment osseux.

10. Prothèse de genou selon la revendication 9, **caractérisée en ce que** le revêtement contient des produits tels que apatite hydroxyle, ou est réalisé à partir d'un titanate-alcool ou d'un alcoxysilane.

11. Prothèse de genou selon l'une des revendications 1 à 10, **caractérisée en ce que** le rayon de courbure (R1) des condyles (3) se réduit au moins une fois dans la zone d'appui depuis la transition de l'extension jusqu'à la flexion complète.

12. Prothèse de genou selon l'une des revendications 1 à 11, **caractérisé en ce que** l'angle α ou β est inférieur à 10°.

13. Prothèse de genou selon l'une des revendications 1 à 11, **caractérisé en ce que** l'angle α ou β est inférieur à 5°.

14. Prothèse de genou selon l'une des revendications 1 à 13, **caractérisé en ce que** des tenons (23) sont ménagés sur les surfaces d'appui (12b) dans la direction d'enfilement, qui facilitent le centrage et l'ancrage primaire.
